# EUROPEAN PATENT SPECIFICATION

(11) **EP 2 598 022 B1**
(45) Date of publication and mention of the grant of the patent: **13.02.2019**
(21) Application number: 10855451.0
(22) Date of filing: 30.07.2010
(51) Int. Cl.: A61B 5/02, A61B 5/0285, A61B 5/021, A61B 5/0295, A61B 5/026, A61B 5/053

(54) **DIAGNOSTIC SUPPORT APPARATUS**
DIAGNOSEUNTERSTÜTZUNGSVORRICHTUNG
APPAREIL DE SUPPORT DE DIAGNOSTIC

(43) Date of publication of application: 05.06.2013
(73) Proprietor: Empirical Technologies Corporation, Charlottesville, VA 22901 (US)
(72) Inventor: BARUCH, Martin, Charlottesville, VA 22901 (US); GERDT, David, Faber, VA 22938 (US); ADKINS, Charles, Earlysville, VA 22936 (US)
(74) Representative: Verhees, Godefridus Josephus Maria
(86) International application number: PCT/US2010/043914
(87) International publication number: WO 2012/015426

(56) References cited:
- JP-A- H11 155 826
- US-A- 5 709 212
- US-A1- 2002 058 876
- US-A1- 2007 016 085
- US-A1- 2007 225 609
- US-A1- 2009 326 386
- US-B1- 6 676 608
- TRACHET B ET AL: "Numerical Validation of a New Method to Assess Aortic Pulse Wave Velocity from a Single Recording of a Brachial Artery Waveform with an Occluding Cuff", ANNALS OF BIOMEDICAL ENGINEERING, KLUWER ACADEMIC PUBLISHERS-PLENUM PUBLISHERS, NE, vol. 38, no. 3, 2 February 2010 (2010-02-02), pages 876-888, XP019786076, ISSN: 1573-9686
- H Olsen ET AL: "Cardiovascular response to acute hypovolemia in relation to age. Implications for orthostasis and hemorrhage", American journal of physiology. Heart and circulatory physiology, 1 January 2000 (2000-01-01), pages H222-232, XP055108843, UNITED STATES Retrieved from the Internet: URL:http://ajpheart.physiology.org/cgi/con tent/abstract/278/1/H222 [retrieved on 2014-03-19]

## Description

### Cross-Reference to related application

NONE.

### Field of the Invention

The present invention relates generally to a system for detection of an abnormal decrease or increase in blood volume, and more particularly to detection of a decrease in the volume of blood plasma.

### BACKGROUND OF THE INVENTION

There have been many attempts to deduce arterial blood pressure from the time-dependent analysis of the arterial pulse, as opposed to an amplitude-dependent analysis, which cuffs and Tonometers, etc. use. The primary advantages of a time based blood pressure monitoring system over one based on amplitude analysis are wearer comfort and inherent calibration.

Amplitude-dependent devices have to couple to the pressure wave within the artery and they have to closely track the coupling force with which they bear down on the artery. The required partial occlusion of the artery frequently leads to distinct skin markings as well as numbness of the hand when the radial artery is monitored, which is the most commonly used site for non-invasive blood pressure monitors. In addition, if the device loses track of the force with which it bears down on the artery, either because of drastic blood pressure changes or because of signal-disrupting movements, it has to be re-calibrated. If this requires inflation of a cuff, such as is the case with the Colin Pilot unit, the wearer will experience additional discomfort.

Previous attempts to deduce blood pressure (BP) from arterial pulse time domain analysis have used the well-known fact that the propagation velocity of the arterial pulse is highly dependent on the arterial pressure. These approaches have used delay times between arterial pulses measured at different arterial sites, such as the brachial and the radial artery pulse sites, or, most commonly have used the time delay between the QRS complex of an electrocardiography (ECG) signal and a pulse measured at an arterial pulse site. In general, such two-site approaches have only been able to track substantial changes in BP using pulse transit time (PTT) but have failed to reliably resolve small changes in BP. An example of a small change in BP that is physiologically important is Pulsus Paradoxus (PP), which is defined as the abnormally large decline in systemic arterial pressure and pulse pressure associated with inspiration, usually due to an airway obstruction such as during an asthma attack.

A further and significant complication in previous PTT measurement approaches has been the determination of the diastolic and systolic BP components. The pulse location in time has usually been determined by establishing a threshold condition near the foot of the arterial pulse, either using a simple percentage of total pulse height rule or other more sophisticated methods, such as the tangent intersection method, which is the intersection of the straight-lines drawn through the rear and the fore-fronts of the arterial pulse wave. Not surprisingly, given the fact that the threshold point is close to the diastolic pressure amplitude range, delay times obtained in this manner have correlated reasonably well with diastolic blood pressure changes. However, two-site measurement approaches have been especially deficient in the measurement of systolic blood pressure variations. This is not surprising because the heartbeat pressure pulse changes dramatically in shape and amplitude as it heads toward the arterial periphery. As a result attempts to compare the time delay evolution of certain points on the pulse measured at different arterial pulse sites, aside from foot-to foot measurements, have been difficult. The changes in pulse shape are due to a number of factors, including changes in the arterial wall material composition that affect the-wall's elastic behavior, the taper of the main arterial branch, the distribution of branch lines, and pulse reflections. The result is that the pulse steepens and contracts, as it propagates.

Background of the invention can be found in the following publications:
Cooke, William H, and Convertino, Victor A, Heart Rate Variability and Spontaneous Baroreflex Sequences: Implications for Autonomic Monitoring During Hemorrhage, J. Trauma, Injury, Infection, and Critical Care, 5-(4):798-805, April 2005.2- Convertino, Victor A, Cooke, William H. Holcomb, John H, Arterial pulse pressure and its association with reduced stroke volume during progressive central hypovolemia, J. Trauma. 2006;61 :629-634.
Davies JI, Band MM, Pringle S, Ogston S, Struthers AD, Peripheral blood pressure measurement is as good as applanation tonometry at predicting ascending aortic blood pressure, J. of Hypertension. 21 (3):571 -576, March 2003
Leonetti P, Audat F, Girard A, Laude 0, Lefrere F, Elghozi JL. Stroke volume monitored by modeling flow from finger arterial pressure waves mirrors blood volume withdrawn by phlebotomy.Clin Auton Res. 2004; 14: 176 -181 .
MacDonald's, Blood Flow in Arteries, 4th ed. Arnold, p. 84, 1998.
Anliker M et.al, Transmission characteristics of axial waves in blood vessels, J. Biomech., 1 , p235-46, 1968

### Summary of the Invention

In accordance with an embodiment of the present invention diagnostic support apparatus is provided which comprises a detecting means for detecting a pulse waveform from a detecting position of an animate being, and measuring means for measuring the time difference between the arrival of a first pulse and the arrival of a second pulse. The first pulse and the second pulse are pulses whose time difference corresponds to blood volume in the animate being, and the first pulse corresponds to the first peak on the pulse wave form and the second pulse corresponds to a different peak on the pulse wave form, in particular, the second pulse corresponds to the third peak.

In accordance with the same embodiment of the present invention the detecting means detects the primary left ventricular ejection pulse and the iliac reflection pulse, the primary left ventricular ejection pulse being the first pulse and the iliac reflection pulse being the second pulse.

In accordance with another embodiment of the present invention, data storage means is provided for storing measured time differences between the first pulse and the second pulse for a period of at least about fifteen seconds and in real time. Preferably the period of time is at least several hours.

In accordance with another embodiment of the present invention data storage means is provided for storing measured time differences for a predetermined continuous period of time sufficient to produce statistically significant arterial pulse parameter data represented by the time difference.

In accordance with another embodiment of the present invention, the period of time can be a plurality of random or predetermined discontinuous time intervals over an extended period of time, such as at least a half hour.

In accordance with still another embodiment of the present invention, a diagnostic support apparatus is provided which includes diagnostic support information creating means to create the diagnostic support information on the basis of the diagnostic support content stored in data storage means and diagnostic support content storage means for storing a plurality of diagnostic support contents for providing diagnostic support. The diagnostic support information creating means comprises characteristic value calculation means for calculating a characteristic value from the time difference between the arrival of a first pulse and the arrival of a second pulse contained in the diagnostic information and creates diagnostic support information based on the characteristic value calculated by the characteristic value calculation means. The apparatus is characterized in that it comprises detection means for detecting changes in the characteristic time differential between the first pulse and the second pulse over a time period sufficient to indicate blood volume changes.

In accordance with another embodiment of the present invention a diagnostic support apparatus includes blood volume indicating means for indicating blood volume by monitoring stored data continuously over the time period and calculating trends in the time differences between the first pulse and the second pulse, wherein increases in the time differences indicate decreasing blood volume and decreasing time differences indicate increasing blood volume.

In accordance with another embodiment of the present invention, a diagnostic support apparatus stores data for a period of time that is at least sufficient to produce statistically significant data to establish an arterial pulse parameter baseline value for blood volume for the animate being and sufficient to show trend changes relative to the baseline value.

In accordance with another embodiment of the present invention, diagnostic support apparatus is provided which has comparison means for comparing the time difference data with historical data indicative of blood volume, the historical data comprising time difference data for the animate being and/or a comparable animate being group. The comparable animate being group is a plurality of animate beings having physical characteristics that correlate with the physical characteristics of the animate being, such as humans of a predetermined age group, of the same type of employment, and/or humans having a predetermined category of physical activity.

In accordance with another embodiment of the present invention, the diagnostic support apparatus further comprising calculating from the time difference measurements a value indicative of progressive central hypovolemia as well as hypervolemia.

In accordance with another embodiment of the present invention a computerized diagnostic support apparatus is provided for generating real time data for use in determining of blood volume and/or blood volume changes that are related to dehydration, blood volume decrease, and blood volume increase in an animate being. The apparatus comprises detecting means for detecting a first pulse and a second pulse, the first pulse and the second pulse being pulses whose time difference corresponds to blood volume in the animate being. The apparatus further comprises generating means for generating time difference data and recording the data in a computer memory data storage means and computer memory data storage means for receiving and storing the data defining the time difference between the arrival of the first pulse and the arrival of the second pulse. Monitor means is provided for monitoring changes in the time difference between the arrival of the first pulse and the arrival of the second pulse. The monitoring comprises determining trends and/or base lines for a person by monitoring for a plurality of intermittent testing intervals of about 10 to 20 minutes.

In accordance with another embodiment of the present invention the diagnostic support apparatus includes comparison means for comparing the time difference data with historical time difference data stored in a computer memory. The historical data comprises time difference data for the animate being, and/or a comparable group of animate beings and the animate being is selected from the group comprising humans and horses.

In accordance with another embodiment of the present invention a diagnostic support apparatus comprises computer memory for storing the historical data, and further comprising generating means for computer generating output data, wherein the output data comprises time data for the time difference between the first pulse and the second pulse for a predetermined continuous period of time. The period of time being sufficient to produce statistically significant arterial pulse parameter data, the data being related to progressive central hypovolemia and/or hypervolemia and computer comparison means for comparing the historical data and the output data.

In accordance with another embodiment of the present invention, the monitoring comprises determining trends and/or base lines for a person by monitoring for a plurality of intermittent testing intervals of about 10 to 20 minutes.

In accordance with another embodiment of the present invention the diagnostic support apparatus comprises diagnostic support information creating means for creating diagnostic support data, information acquisition means for acquiring created diagnostic information concerning at least one patient, diagnostic support content storage means for storing a plurality of diagnostic support contents for providing diagnostic support, transmission means for transmitting the stored diagnostic support content, and diagnostic support information display means for displaying the diagnostic support information created by the diagnostic support information creating means.

It should be recognized that reflected pulses readily propagate through the arterial system, and the pulse measured at a certain arterial site is actually a superposition of a number of different and distinct pulse components. Therefore, knowledge of these pulse components and how they travel through the arterial system as a function of blood pressure is essential to make meaningful pulse time delay measurements for the purpose of blood pressure determinations. In the absence of a comprehensive physical understanding of the structure of the pulse in the arterial periphery it is therefore not surprising that commercially viable time- domain analysis approaches of the arterial pulse have so far limited themselves to the determination of arterial pulse propagation velocities alone.

The present invention avoids the problems and disadvantages of multiple site blood pressure measurements provides single-site measurement of blood pressure with less complexity and lower cost than has heretofore been possible. It has now been discovered that a well-known pressure-velocity relationship that has been shown to hold for pressure-change induced pulse propagation changes also holds for the components of a single arterial pulse. In addition it has been determined that the component pulses of which the arterial pressure pulse is comprised, can be distinctly determined.

Knowledge of where these component pulses originate, what arterial distances they have traversed, as well as their measured relative time delays (and their measured amplitudes relative to the primary systolic peak)) makes it possible to determine the blood pressures, both systolic as well as diastolic, that influenced their relative delay times.

In contrast with the foregoing systems, a time-based arterial pulse analysis approach is less dependent on the coupling pressure to the arterial pulse. As long as the sensor is linear as well as sensitive enough to record the entire arterial pulse shape with high fidelity, it is possible to deduce from the time evolution of the arterial pulse the blood pressure to which the pulse is subjected. Since such a device does not have to couple to the artery's pressure wave as aggressively, wearer comfort is increased. In addition, by using algorithms that are based on a physiological model of the arterial pulse, the approach is neither subject to continued re-calibrations after motion has occurred, nor otherwise induced disruptions of the signal. This is due to the fact that a time-based arterial pulse analysis approach constitutes tracking the time evolution of physiologically relevant markers in the arterial pulse. As long as the algorithm reacquires the time positions of the relevant markers, the original calibration that linked diastolic and systolic as well as mean blood pressure components to the time markers will hold. The goal has been somewhat elusive up until now because of the uncertainty of determining physiologically relevant arterial pulse markers.

In accordance with a first broad aspect of the present invention blood pressure (BP), and more particularly non-occlusive, passive blood pressure is measured using a sensor of heartbeat pulses at a single site and with a resolution sufficient to resolve small variations in blood pressure. The invention utilizes a primarily time-dependent pulse wave analysis that is based on a physiological model of the components of the arterial pulse. In accordance with a further aspect of the present invention, the problems due to different pressure-induced pulse-shape modulations associated with different pulse detection sites are avoided by detection of single heartbeat pulses at a single site and by analysis of individual pulses.

It has now been found that time-dependent pulse wave analysis can be used to provide diagnostic support for detecting dehydration or hemorrhaging. The system of the present invention comprises means for monitoring the time difference between the arrival of the primary left ventricular ejection pulse (pulse T1 ) and the arrival of the iliac reflection (pulse T3) to determine an arterial pulse parameter which is the time difference between T1-T3. Decreasing the time differences is related to decreases in blood volume and increases in the time differences are related to increases in blood volume detection.

In regard to another aspect of the invention it has now been found that changes in T3 -T1 are indicative of something happening to blood volume. If the T1 -T3 value goes up and the patient is on an infusion system, it can be an indication of having too much fluid being infused and if T1 -T3 is lower than it should be for an individual, it can provide information that can be used to indicate that they are either dehydrated (which can result in decreases in blood volume), they are hemorrhaging, or they have hemorrhaged. A downtrend in T13 can tell whether someone is continuing to hemorrhage. Measurement of the change of the parameter T1 -T3 is carried out in real time. T13 values that are low in comparison to the values for a comparable patient group, such as a particular age group, could indicate with a very short reading that the patient has either had a blood loss or was dehydrated.

### Brief Description of the Drawings

Figure 1 is a graph illustrating the five constituent pulses that make up the finger pulse;
Figure 2 is a second graph of the five pulses used to constitute a finger pulse;
Figure 3 is a drawing of the arteries involved in creating the pulses of Figures 1 and 2;
Figure 4 is a graph illustrating an overall change in heart rate as a function of lower body negative pressure (LBNP) for 15 subjects.
Figure 5 is a graph illustrating the evolution of heart rates for two subjects of the entire course of their LBNP session showing that while subject #5's heart rate responds strongly, and subject #9's heart rate responds negligibly.
Figure 6 is a graph illustrating overall study results for systolic and diastolic blood pressures obtained with the automatic cuff.
Figure 7 is a graph illustrating overall results for the P2-P1 ratio, the PDA parameter that is equivalent to' systolic pressure, as a function of LBN P.
Figure 8A is a graph illustrating overall results for pulse pressure obtained with the automatic cuff;
Figure 8B is a graph illustrating overall results for pulse pressure obtained with the PDA pulse pressure-equivalent parameter T1 -T3. Also shown are results of second order polynomial fits.
Figure 9 is a graph illustrating the comparison of the individual results for cuff based pulse pressure and PDA based T1-T3 measurements for subjects 3 - 5.
Figure 10 is a graph illustrating fifteen- second averages of T1 -T3 values obtained within a minute of the cuff measurement.

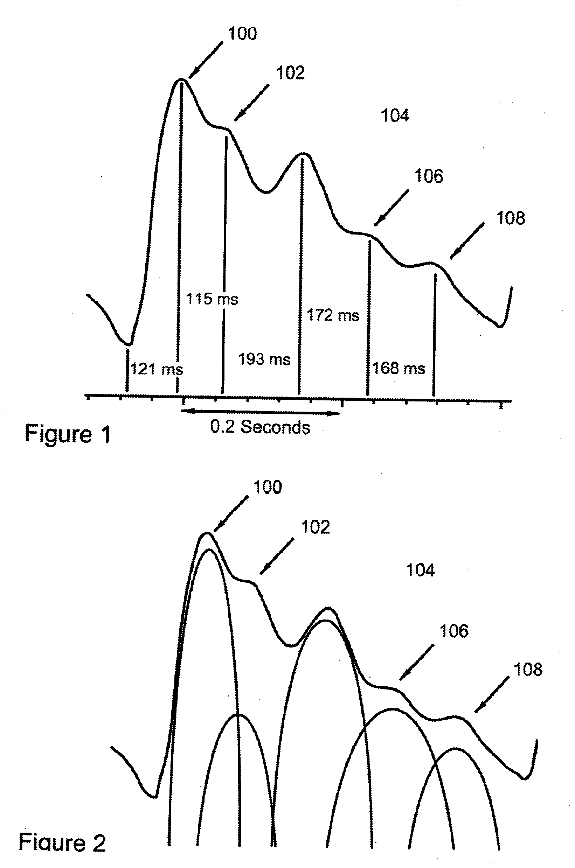

Figure 1 1 is a graph illustrating the correlation between T1-T3 PDA parameter and pulse pressure.

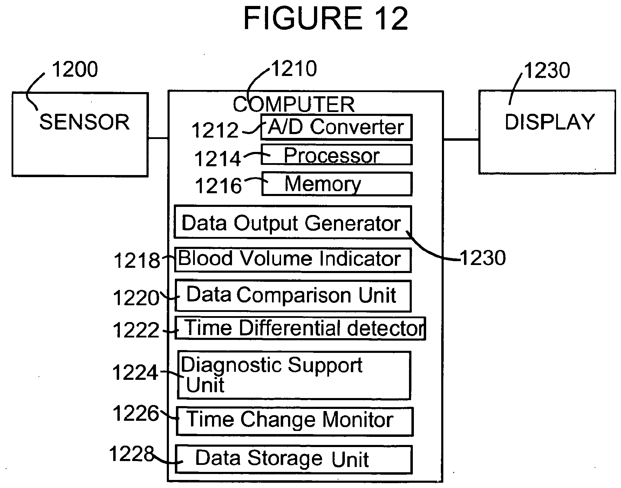

Figure 12 is a schematic illustration of an apparatus in accordance with the present invention.

### DESCRIPTION OF PREFERRED EMBODIMENTS

### Definitions

Where the definition of terms departs from the commonly used meaning of the term, applicant intends to utilize the definitions provided below, unless specifically indicated.

For the purposes of the present invention, the term "plethysmograph" refers to an instrument that measures variations in the size of an organ or body part on the basis of the amount of blood passing through or present in the part.

For the purposes of the present invention, the term "horse race" refers to a contest of speed among horses that either are ridden by jockeys or pull sulkies and their drivers.

For the purposes of the present invention, the term "substantial" refers to an ample or considerable amount, quantity or size. Accordingly, the term "substantially comparable" as employed herein refers to data or information that can enable a diagnosis to be made based on a comparison of the patient's real time data to the substantially comparable data.

For the purposes of the present invention, the term "Valsalva episode" or "Valsalva maneuver" refers to the expiratory effort when the mouth is closed and the nostrils are pinched shut, which forces air into the Eustachian tubes and increases pressure on the inside of the eardrum, and to the expiratory effort against a closed glottis, which increases pressure within the thoracic cavity and thereby impedes venous return of blood to the heart. Essentially, Valsalva maneuver is any attempted exhalation against a closed glottis or against a closed mouth and nose.

A Valsalva maneuver performed against a closed glottis results in a drastic increase in pressure in the thoracic cavity, the airtight section of the torso that house the lungs and heart. In normal exhalation, the diaphragm relaxes, pushing up and into the thoracic cavity. This increases pressure in the cavity and forces the air out of the lungs. However, when the air cannot escape, as when the glottis is closed in a Valsalva maneuver, pressure simply continues to build inside the thoracic cavity until the diaphragm relaxes or the air is allowed to escape. This reduces the amount of blood flow into the thoracic cavity, especially in the veins leading to the right atrium of the heart.

For the purposes of the present invention, the term "interbeat interval" refers to the time interval between temporally adjacent heartbeat pulses.

For the purposes of the present invention, the term "monotonically" refers to the designating of sequences, the successive members of which either consistently increase or decrease but do not oscillate in relative value. Each member of a monotone increasing sequence is greater than or equal to the preceding member; each member of a monotone decreasing sequence is less than or equal to the preceding member.

The system of the present invention uses a pulse decomposition analysis (PDA) and algorithm to determine the changes in both the systolic and diastolic pressure thereby enabling a non-invasive monitoring system for the purposes of predicting the onset of medical conditions. A battery operated fourteen ounce unit tracks systolic, mean and diastolic blood pressure, beat by beat, wirelessly sending the raw data to a PC. Initial values of blood pressure are entered by manual measurement or automatic cuff system. All data is analyzed at the user's PC, supplying, plotting and storing results in real time.

Although prior art has used cuffs, the readings obtained have never been able to be used as a determinate of blood pressure changes. The algorithm used in the system of the present invention monitors the time difference between the arrival of the primary left ventricular ejection pulse (pulse #1 ) and the arrival of the iliac reflection (pulse #3) to determine an arterial pulse parameter T1 -T3. T1 -T3 may also be referred to herein as T13 or T1-T3. TheT13 differential enables system to determine, the onset of conditions such as hemorrhagic shock, blood loss, as well as monitor blood transfusions.

The basic components of the algorithm are 1 . a peak finder that identifies heartbeats in the derivative data stream, 2. a differentiator that produces the second derivative of the detected heart beat which is then used to find the inversions corresponding to the locations of the component pulses, 3. a digital integrator, implemented as a Bessel filter, that generates the integrated pulse wave form from the differentiated raw signal stream, and from which relative component pulse amplitudes are determined and 4. a low-pass filter that allows identification of the primary systolic peak. Furthermore the frequency content of the data stream is continuously analyzed in order to calculate signal to noise (S/N) figures of merit that determine whether signal fidelity is sufficiently high to permit peak detection and analysis.

Once the temporal locations of the reflection component pulses and the systolic peak are identified, the T13 interval, the time delay between systolic (P1 ) and iliac peak (P3), is calculated. The P2P1 ratio is calculated using the amplitudes of the P2 peak and the systolic peak, in the integrated pulse spectrum.

Before describing the details of the invention it is necessary to provide an overview of the physiological model that underlies the approach of the invention. The benefit of the model is that it provides a physiological understanding of the structure of the arterial radial pulse as a result of which arterial pulse analysis algorithms can be developed based on a physical model of the arterial tree, as opposed to for example, implementing a multi-variable mathematical model that correlates newly acquired pulse shapes with a large set of previously stored pulse shapes, or using a generalized transfer function to reverse the filtering effect of the arterial tree on the propagating arterial pulse. While the description given here is limited to applying the model to the radial arterial pulse, it will become clear in the context of the description of the model that it can readily be extended to other pulse sites.

### A MODEL OF THE RADIAL PULSE

At the core of the model is the concept that the radial arterial pulse is a superposition of several component pulses. At the temporal front of the radial pulse envelope is the primary pressure pulse that results from the contraction of the left ventricle and the subsequent ejection of blood into the arterial system. Additional component pulses give rise to the temporal features of the radial arterial pulse that follow this primary pulse. Isolation and identification, with regard to time and amplitude, of these individual component pulses provides an analysis from which information about blood pressure as well as arterial tree health can be obtained.

### Background

A basic understanding of the physical circumstances of the propagation of the arterial pulse from the heart to the periphery was achieved decades ago. The picture is one of an arterial pressure pulse that originates at the interface of the left ventricle and the aortic root traveling away from the heart through the arterial tree and being reflected at various sites to various degrees. The reflection sites are areas where the arterial tree branches or where different diameter sections join. Both types of sites present an impedance mismatch to the propagating arterial pulse, giving rise to reflections. The existence and the physiological consequences of reflections in the arterial tree are now commonly accepted. One example is the "diastolic wave" which is clearly a reflection phenomenon. In young and elastic arterial trees this reflection arrives back at the heart well into the diastolic phase of the cardiac cycle and has the beneficial effect of raising the blood pressure outside the closed left ventricle, thereby enhancing perfusion of blood into the coronary arteries. As the arterial tree ages and hardens, pulse velocities increase and reflections arrive earlier. Pathologies arise when the reflections arrive while the left ventricle is still open. The heart now has to contract harder to overcome the additional pressure in the aortic root, leading to wall thickening and other complications. Also, since the pressure in the aortic root is now lower during the diastolic phase, perfusion of the coronary arteries is diminished.

The above description of the existence of reflections and their physiological impact is well established in the medical literature. Extensive clinical studies and theoretical modeling efforts have been performed to investigate various aspects of arterial pulse reflections, such as the "second systolic peak", yet no clear model with regard to the radial arterial pulse has been proposed as to where exactly the reflections arise. As an example, an asymmetric T-shaped model where the pulse originates at the T junction and the ends of the T represent generalized reflection sites of the lower body and the upper body has been proposed. The model does a reasonable job in explaining the shape of the aortic pulse that has been analyzed in detail in a number of clinical studies but it draws no conclusions about what effect these findings should have on the shape of the pulse in the arterial periphery, such as the radial pulse.

It is not necessarily reasonable to assume that there are distinct reflection sites in the arterial tree as opposed to the assumption that, as an example, "the lower body" as a whole gives rise to the reflections that have such physiological significance to cardiac health? The answer is two-fold. One is that the features of the reflected wave are too distinct, and too sharp, as to be the convolution of different reflections originating from different sites with different time delays and different reflection coefficients, which would tend to broaden out specific pulse features. The second answer is that the arrival times of the specific features of the radial pulse very much narrow the location possibilities of the reflection sites that gave rise to them.

One feature almost all of the radial pulse signatures share is the fact that they exhibit pulse like protrusions that have a time duration comparable to that of the primary pulse. One consequence of Valsalva is the shortening of the cardiac ejection period as a result of which it is possible, in a comparatively young and elastic arterial tree, to see the complete separation of primary pulse and reflected pulse. As observed the reflected pulse shows no broadening compared to the primary systolic peak, supporting the hypothesis that it originated at a distinct refection site.

While a distinct reflection site will give rise to a reflection that bears strong resemblance to the primary pulse, distributed and multitudinous reflection sites will give rise to a plethora of reflected pulses, arriving at different time delays and with different amplitudes. The superposition of such a system of reflection sites will be a featureless, broadened pulse. The presence of distinct pulse-like features in most of the radial signatures shown therefore suggests that, past the primary systolic peak, distinct reflection sites are responsible for the sequence of reflected pulses that comprise the "diastolic wave". While the presence of distinct pulse-like features in the radial pulse suggests the existence of distinct reflection sites, their time of arrival relative to the primary pulse makes the argument significantly more concrete.

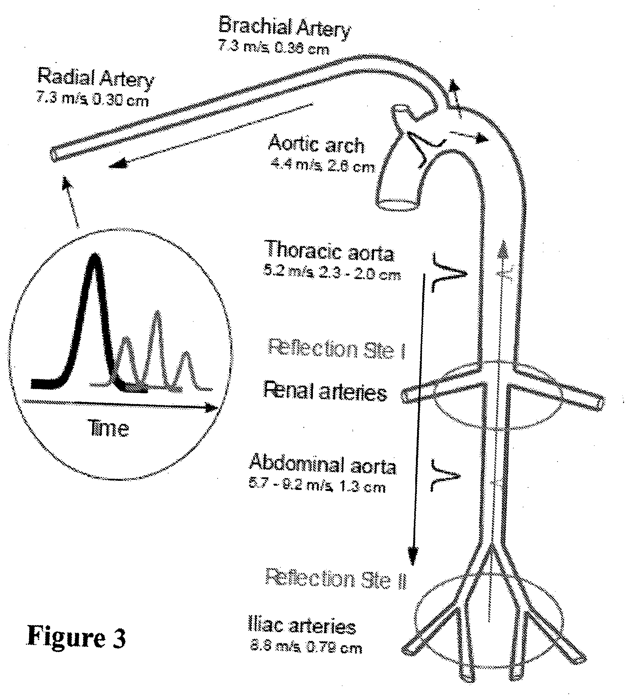

Since arterial pulse propagation velocities have been measured throughout the body, it is possible to match time delays with potential reflection sites. If one uses approximate arterial distances and their respective velocities, the "second systolic" peak matches readily with the site labeled "reflection site I" while the third peak matches with "reflection site II" as shown in Figure 3.

In 1985 Latham performed a detailed experimental study to map out the shape of the pressure pulse in the different sections of the aorta using a specially designed catheter with spaced micronanometers. His work clearly demonstrated the existence of two major reflection sites to the down-ward traveling arterial pulse, one being in the region of the renal arteries, the other beyond the bifurcation of the iliac arteries. At the location of the renal artery the diameter of the aorta, which tapers continuously away from the heart, undergoes its greatest change. This discontinuity presents a significant impedance mismatch to the traveling pressure pulse, as a result of which an appreciable part of its amplitude is reflected. The reflection can be reduced using the Valsalva maneuver, which involves exhaling into closed airways. As a result of the increasing pressure within the thoracic cavity the diameter of the thoracic aorta decreases (on the order of 17% as Latham verified ultrasonically). The maneuver therefore alleviates the aortic diameter change at the renal arteries, which reduces the impedance mismatch, thereby lowering the site's reflection coefficient.

Latham also found a second reflection site beyond the bifurcation of the iliac arteries, the contribution of which to arterial pulse reflections in the aorta was ascertained using manual femoral artery occlusion maneuvers. Other contributions to the tail end of the aortic pulse were attributed to diffuse arterial pulse reflections from the periphery. In view of Latham's work it therefore seems very likely that the two peaks visible past the systolic peak originate at the reflection sites indicated. Valsalva experiments performed as part of this work further support the model.

The next peak in the radial pulse, that is, the "diastolic peak", as well as the peaks that follow likely arise from the iliac arteries reflection site and not, as Latham had proposed, due to diffuse reflections from the arterial periphery. Latham's explanation with regard to the structure appears to be unlikely, given the distinct peak structure with spacing comparable to that of the "second systolic" and the "diastolic" peak.

Furthermore, the time delay of such reflections would extend up to 250 ms past the "diastolic" peak if some of them truly traversed the length of the legs. Indeed, recent work supports the hypothesis that the peaks visible past the "diastolic" peak are in fact due to re-reflections between the two reflection sites, a reasonable proposition given the strength of the sites' reflection coefficients (10 - 15% in the case of the renal arteries reflection site, up to 30% in the case of the iliac arteries reflection site).

J. Kriz et. a/, showed that it is possible to use force plate measurements as a noninvasive method to perform ballistocardiography, the motion of the body associated with heart activity, by displaying the motion of the heart muscle and the subsequent propagation of the pulse wave along the aorta and its branches. With subjects lying horizontally on a bed that was placed on a force plate they were able to identify the ground reaction forces arising from such center-of-mass altering events as the heart muscle contraction as well as the resulting blood pulse flow. The resolution of the apparatus was sufficient to clearly resolve events involving the redirection of momentum of the propagating arterial pulse, such the pulse's traversal of the aortic arch, its partial reflection at the renal artery site, the iliac reflection site, as well as the subsequent re-reflections of the reflected pulses. As an aside, in subjects with an aortic aneurism, the site of the arterial distension was clearly identifiable due to its effect on the neighboring "normal" reflection sites.

The basic model of the radial arterial pressure pulse is therefore one of a convolution of the primary systolic peak, its single-pass reflections from the renal arteries and iliac arteries reflection sites, as well as their double-pass re-reflections.

In order to understand the details of this time delay contraction, one has to be able to determine the arrival times of the individual component pulses at the wrist independently of each other, that is, an "external" clock, as opposed to one started at the onset of a given radial pulse, is required to time the separate arrivals. One means of establishing an "external" clock is to use an ECG signal relative to which the arrival time of each component pulse at the radial artery is measured. Using the Colins Pilot tonometric blood pressure monitor, a subject's ECG and blood pressure was collected in addition to the wrist sensor signal in real time during periods of rest and during the course of a Valsalva maneuver. As one would expect, the oscillations in the delay time of the #1 pulse mirror the pressure oscillations. This is to be expected since pulse travel time and pressure are inversely related. In contrast to the #1 pulse, the delay time of the #2 pulse is far steadier, showing no obviously matching modulations. This is also to be expected because the #2 pulse, after traveling to the renal reflection point at systolic pressure, returned as a reflection at a much lower pressure. It also traversed only the softest part of the aorta, the section above the renal reflection point. Consequently, its velocity will be least affected by arterial pressure changes. In line with this, one would expect the #3 pulse to exhibit a higher sensitivity to changing blood pressure environments. From the Kriz experiments it appears that the iliac reflection is a far more pronounced reflection site than the renal site, as a result of which the #3 peak is also usually significantly larger in amplitude than the #2 peak in the radial arterial pulse spectrum. Consequently, the #3 pulse, which on its primary path to the iliac reflection site, traversed the stiffer and therefore faster abdominal aorta as well as the fast iliac arteries, and returns as a reflection at a higher pressure and therefore higher velocity, compared to the #2 pulse. Traveling at a higher pressure subjects the #3 pulse, similarly but not quite as strongly as the #1 pulse, to the steeper part of the arterial non-linear relationship between pressure and velocity.

Another subtle but very important detail is visible in the evolution of the arrival times of the component pulses during the Valsalva maneuver. The #3 pulse responds first to the rising pressure at the onset of Valsalva. Visual inspection establishes readily that both the arrival time of the #1 pulse as well as the BP line shapes measured with the Colins monitor move off their baseline well after the marker while the arrival time of the #3 pulse has responded well before (approximately 4 seconds before the Colins signals and the #1 component pulse). The delayed reaction of the Colins signals and the #1 component pulse relative to the response of the #3 pulse is a result of the different Young's moduli of the involved arteries. In the absence of significant hardening of the central arteries (the subject in this case is a 46 year old runner in fit shape), the arterial walls in the arm, and in the arterial periphery in general, are significantly tougher than those of the central arteries, a well-known fact due to different elastin versus collagen content in the walls. Since a given rise in blood pressure will tend to distend the softest sections of the arterial tree first, it is entirely reasonable to expect the pulse propagation velocities of the central arteries to also increase first. Consequently one would expect the #3 pulse, which samples the entire aortic tree twice along its propagation path, to accelerate relative to the #1 pulse, which traverses essentially only the arm complex arteries that are characterized by significantly less compliant wall material. The same reasoning explains the time delay between the response of the #3 pulse and the onset of the Colins monitor, which measures its signal at the radial artery.

How the time delay between the #1 and the #3 pulse evolves as the pressure continues to rise is also determined by the differential Young's moduli of the arm and central arteries. In persons with "elastic" central arteries one observes the continued narrowing of the time delay between the #1 and the #3 pulse with rising pressure, indicating that propagation velocities of the central arteries, due to their significantly higher distensibility, continue to change faster than those of the arm complex and the arrival time of the #3 pulse changes faster due to the much longer path length over which velocity changes can manifest themselves. In persons with "hard" central arteries, the time delay between #1 and #3 is markedly different. In the case of "hard" central arteries the time delay between #1 and #3 increases with rising blood pressure. Since in this case the central arteries have very little excess distensibility relative to the arm, or peripheral, arteries, the arm arteries respond equally to a rise in pressure. However, due to the higher pulse velocity propagation and the higher gain of the pulse propagation velocity as a function of pressure in the arm versus the central arteries, the #1 pulse continues to accelerate away from the #3. Remarkably, it is possible to observe an intermittent state of the evolution of the delay time between #1 and #3 in the same patient, that is, in the presence of continuously rising pressure, the delay time initially decreases, reverses, and then continues to increase.

Clearly such patients have only some hardening of the central arteries as a result of which they exhibit the pressure onset behavior of patients with "elastic" arteries. The limits of "easy" distensibility are, however, quickly reached and the pressure load is increasingly shared by the peripheral arteries, and specifically those of the arm as a result of which, for the same physical reasons that were given above, they exhibit the delay time behavior of "hard" artery patients at higher pressures.

Returning once more to the case of persons with "elastic" central arteries, the reversal of the delay time between #1 and #3 with increasing blood pressure may also occur in this case, but at a much higher pressure. While the time evolution of T13 (time delay between pulse #3 and pulse #1 ) as well as the relative amplitude of P3 and P1 is comparatively straightforward, the time delay and amplitude evolution of the pulse relative to the P1 pulse is somewhat more complex. This is due to the fact that the P2 pulse has an additional degree of freedom relative to the P1 and P3 pulses in that its amplitude relative to the other two pulses changes with blood pressure, specifically pulse pressure. This point is perhaps more clearly made after first examining the amplitude evolution of the P3 and P1 peaks as a function blood pressure, specifically systole. The P3 pulse arises from the reflection site in the vicinity of the iliac arteries. This reflection is due to a combination of effects due to arterial bifurcations as well as changes in arterial diameter. Ageing effects, such as through the deposition of plaque, will also alter the reflection site, but these are long term and slowly-varying effects. In contrast, the physical parameters of this reflection site are not likely to change appreciably with blood pressure. Put differently, the reflection coefficient of the site is not very pressure dependent. Therefore, if the amplitude of P1 increases because the systolic blood pressure has increased relative to the diastolic floor, P3 should increase proportionally, or the ratio of P3/P1 should remain largely constant with changes in blood pressure. Observations to date have shown this to be the case.

In contrast to the amplitude response of P3, which maintains its proportionality to P1 , the ratio of amplitudes P2/P1 increases proportionally with blood pressure. This is not surprising since the fact that the "second systolic peak" becomes very prominent in cases of high blood pressure is well known and readily observable. The P2 pulse arises from the reflection site at the height of the renal arteries that is characterized by a diameter mismatch between the thoracic and the abdominal aorta. With increasing blood pressure the thoracic aorta's diameter increases and it does so at a faster rate than the abdominal aorta due to a difference in wall material strength. Consequently, the amplitude of the P2 pulse will increase at a different rate than the P1 with increasing blood pressure, that is, the ratio P2/P1 will increase. The increased amplitude of the P2 pulse will also modify its propagation velocity, which depends highly on the pulse's amplitude. The resulting non-linear delay time behavior, which is due to the fact that the pulse increasingly accelerates as its amplitude rises, can be observed in large amplitude blood pressure variations such as are observed in dialysis patients.

A final consideration that completes the description of P2's temporal and amplitude evolution is the fact that its amplitude is actually proportional to pulse pressure, that is, the difference between systolic and diastolic pressure. This of course is also the case for P3, since it is only the pulsatile part of the blood pressure that can produce a reflection. In the case of P2, however, the fact that its amplitude changes relative to the amplitude of P1 gives rise to the interesting opportunity that the ratio of P2/P1 is a measure of the pulse pressure, self-referenced within each heartbeat pulse and therefore largely independent of coupling efficiencies.

While the pulse pressure is determined using T13, the diastolic pressure is tracked by monitoring the ratio of the amplitudes of the #2 and the #1 pulse, i.e. P2/P1 , which rises monotonically with pulse pressure. The starting values for correlating P2/P1 with pulse pressure are however very different for different patients since the ratio is small (on the order of 0.04) for patients with "hard" central arteries, and larger (0.2) for patients with "elastic" central arteries at comparable normal blood pressures. Patients with hard central arteries tend to have, at normal blood pressures, diminished P2 amplitudes, which increase dramatically with rising blood pressure. Patients with "elastic" central arteries tend to have very pronounced P2 amplitudes at resting blood pressures, indicating that their thoracic aortas are significantly more distended than patients with "hard" central arteries at comparable blood pressures. This observation is supported by published results that demonstrated a drop in aortic pulse propagation velocities by about 10% in subjects who changed from a sedentary lifestyle to one characterized by endurance exercise training. The effect, which was demonstrated to be entirely reversible with cessation of exercise, was shown to be due to a change in aortic distensibility.

With the blood pressure extremes determined, the mean arterial pressure is then determined by obtaining the ratio of the integral over the line shape of the full radial arterial pulse to the time interval over which the integral is performed, a standard procedure. It is clear from the above example and the previous discussion of the influence of the reflection sites on the component pulse amplitudes that, by comparing ratios of the relative amplitudes of the three (or more) component pulses, the relative magnitudes of the renal and iliac reflection site coefficients, or RFL2 and RFL3, can be determined. The reflection coefficient associated with the interface between the arterial junction between the aortic arch and one of the subclavian arteries, RFL 1 , has to be determined independently and in the present analysis it has been simply set to 10%. However, its influence on the analysis is minimal since its effect is common to all pulse paths. In addition the RFL 1 coefficient is, similarly to the reflection coefficient RFL3 associated with the iliac reflection site, not likely to change except over significant time frames that allow for relatively slow physiological processes such as, for example, the deposition of atherosclerotic plaque to take place. It is also clear that the effectiveness of implementing the model presented above depends entirely on the efficiency of the algorithms that are used to detect a. the individual radial heart beat pulses and b. the composite pulses that comprise the radial pressure pulse shape. One approach to detect the heartbeat pulses as well as the composite pulses will now be described in detail. It is understood that a plethora of different approaches are available to accomplish the same tasks.

Figures 1 and 2 are graphs that illustrate the five constituent pulses that make up the finger pulse in an example pulse waveform taken on a finger using the instant system. In this example, the primary systolic peak T1 100, renal reflection peak T2 102, iliac reflection peak T3 104, re-reflection peak 106 and re-re-reflection peak 108 are illustrated. P2/P1 indicates the relative amplitude of P2 track systolic blood pressure and T1 ,3 is the time between the two pulses T1 100 and T3 104. It should be noted that pulse creating T1 100 from the left ventricular ejection, travels at a velocity in the systolic pressure regime while the pulse of T3 from the iliac reflection travels at a velocity closer to the diastolic pressure regime. Figure 3 is a drawing of the arteries that are involved in the pulses of Figures 1 and 2.

An algorithm for use in the present invention is disclosed in US Patent 7,087,025, entitled, "Blood pressure determination based on delay times between points on a heartbeat pulse", which is incorporated herein by reference, as though recited in full.

T1 -T3 can also be found from other algorithms or methods, which could include hard wired circuits and no software. The key point is finding the center of the primary peak (in time) and the center of the iliac reflection (third peak, second reflection). Once found, both are simply time in milliseconds. Changes in T3-T1 are indicative of something happening to blood volume.

Automatic infusion pump systems will require blood pressure measurement as near to continuously as possible. The fastest of conventional, automatic arm-cuff systems measures systolic blood pressure every one or two minutes which takes a minimum of 15 seconds per measurement. This load on the arm is not pleasant after a short time. In the fastest conventional systems, the power requirements are high because the pumps are large and powered for high duty cycles. The disclosed system measures blood pressure every heartbeat for over 12 hours on a cell phone battery and causes no discomfort. If the T1 -T3 value goes up and the patient is on an infusion system, it would be an indication of hypervolemia, too much fluid pumped in (very dangerous especially in the elderly, because it blows out organs). If T1 -T3 is lower than it should be for an individual, they are either dehydrated, they are hemorrhaging, or they have hemorrhaged. The trend in T1 -T3 can also indicate whether someone is continuing to hemorrhage.

Although not always necessary, the monitoring of T1 -T3 frequently provides the greatest value when performed in real time. "Real time", as used herein, refers to the actual time that it takes a process to occur. In the present system information/data is updated in real time. In the area of computer science, the term "real time" refers to the time it takes for a process under computer control to occur. In computer systems information is updated at the same rate they receive the information, that is, immediately.

In one embodiment of the invention, the system of the present invention operates in real time to measure T1-T3 over a period of time that is sufficient to establish changes or trends in T1 -T3 over an extended period of time. The time periods of the testing as well as the intervals between tests can vary depending on the condition being monitored and the protocol of the parties monitoring. In a hospital some protocols will call for measuring beat by beat, for example during major surgery, while others will measure over a predetermined time period. Trends and base lines for a person can be obtained after a few periods, or intervals, however generally the length of the testing period is at least about fifteen (15) seconds.

For example, intermittent testing can be taken, approximately at 15 minute intervals. The time period for the testing can range from a few minutes to a half hour, with 10 to 20 minutes being preferred range for certain applications. The intervals for testing in a battery operated system can also be based on the battery life in addition to patient condition.

A critical factor is the establishment of a baseline so that deviations, such as caused by either blood loss or dehydration, can be recognized. Deviations from established baseline will have more meaning as the individual's base line is known with greater accuracy. Dehydration produces the same reading as internal or external blood loss as it also results in decreased blood volume.

A baseline could be established either by 15 seconds of continuous measurements or by the equivalent number of heart beats obtained at discontinuous times, say at random time intervals over an hour. It is likely that T13 will not change appreciably, except under extreme conditions, for an individual except over many years.

In another embodiment of the invention, the system of the present invention measures T13 for a particular patient and compares the T13 to known values (also referred to as historical data) for a comparable patient group. A comparable patient group, as employed herein, refers to a group having features in common with the patient undergoing monitoring. The features can be equivalent, or at least sufficiently similar to be worthy of comparison and can include age, physical build, similarity of employment, life style, general health, etc. Data of a comparable patient group can be relied upon to prove data sufficiently similar or equivalent to that of the patient to enable a diagnosis to be made in the absence of, or in addition to a patient's personal historical data.

### Applications of the system of the present invention

With respect to uses of the system of the present invention, in the case of marines or soldiers, when necessary the system can be used without obtaining a baseline. Generally soldiers or marines are fit, and fall within a similar age and weight range and therefore T13 is likely similar with all of them, somewhere a little above 300 msec. For an individual who had an established T13 baseline of about 300 and then had one measurement showing a T13 of 250, it could be determined that either they are dehydrated or they have lost a lot of blood. In instances where there is no individual baseline, demographics can be relied upon. T13 values that are low for a particular age group could indicate with a very short reading that this person either had lost blood or was dehydrated. It is known that thoroughbred horses have bleeding lungs after a race. The disclosed system has been used to measure pulse patterns in horses and has achieved the same results as with humans using the T13 deviations as an indicator to hemorrhaging.

Dehydration is probably the most important parameter for troops in many countries such as Iraq and Afghanistan however there is no good way to measure it.

Dehydration is also a problem in horses, especially work and race horses, and although you can pinch their necks and see how long the pinch marks take to go away; this procedure provides a very approximate test. Using the disclosed system, dehydration can be determined by monitoring the deviation from the baseline of either the individual, if known, or a comparable group.

A quick test, using the disclosed system, in the absence of dehydration, can be conducted to see if someone has hemorrhaged. Longer term measurements can be used to monitor the rate of hemorrhaging.

Dengue fever can get very serious if and when hemorrhaging starts (dengue hemorrhagic fever). Most health providers feel the stomach to see if it is hard like wood; however, at that point, it is very late for treatment with anticoagulants. Dengue fever outbreaks have occurred worldwide and there have been reported cases in Florida and Texas creating a concern about dengue hemorrhagic fever in the United States. The system of the present invention can be used to monitor a patient for hemorrhaging, and is particularly useful in regard to dengue hemorrhagic fever because it is a non-invasive test that can be used to monitor a patient for extended periods of time, as for example, for hours, days, etc.

### Experiments/Testing

Tests of the system of the present invention were performed at the Cardiovascular Physiology and Rehabilitation Laboratory of the University of British Columbia on fifteen healthy volunteers (average age: 24.4 years, SD: 3.0 years; average height: 168.6 cm, SD: 8.0 cm; average mass: 64.0 kg. SD: 9.1 kg) whose lower bodies, from the height of the navel down, were subjected to increasingly negative pressures. Lower body negative pressure (LBNP) is an established technique used to physiologically stress the human body, particularly the cardiovascular system. LBNP is used to simulate gravitational stress, to simulate hemorrhage, alter preload, and to manipulate baroreceptors. A number of studies have demonstrated that it is possible to simulate significant internal hemorrhage using LBNP. Negative pressures of 10-20 mmHg correspond to 400 to 550 ml of central blood loss, 20-40 mmHg correspond to 500 to 1000 ml, and negative pressures in excess of -40 mmHg correspond to blood losses exceeding 1000 ml. See publication 1 for background.

The subjects were subjected to four stages of negative pressure, -15 mmHg, - 30 mmHg, -45 mmHg, and -60 mmHg, each stage lasting typically about 12 minutes. The blood pressure was monitored with an automatic cuff (Bp TRU Automated Non- Invasive Blood Pressure Monitor (model BPM-100), VSM MedTech Devices Inc.) set to record blood pressures every three minutes, resulting in typically four readings per LBNP setting, and a pulse oximeter (Ohmeda Biox 3740 Pulse Oximeter, BOC Health Care) monitored oxygen saturation. The System of the present invention collected arterial pulse shapes via a finger cuff attached to the central member of the middle digit. Four subjects became presyncopal and could not complete the -60 mmHg LBNP stage. Both real-time as well as statistical results in the form of regressions are presented. Statistical data are presented as means +- standard error.

### Results Heart Rate Changes

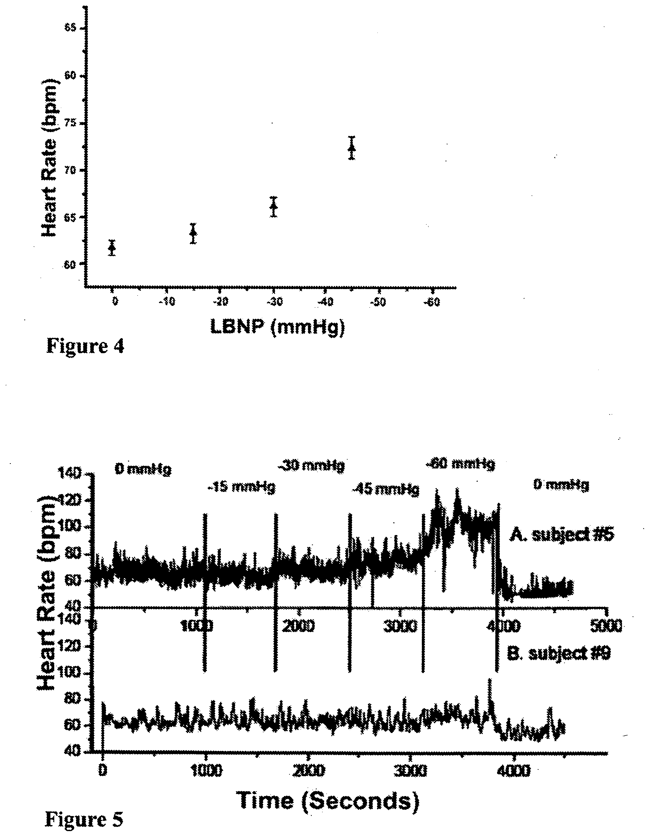

Most of the subjects responded with significant increases in heart rate to the increasing negative pressure. Figure 4 presents the overall means of heart rates obtained with the system of the present invention for all fifteen subjects. The average effect is clearly resolved, a result that has been verified by other investigators. See for example, publication 2. It is however also well known that heart rate is of limited value as a determinant for the onset of hemorrhage. Figure 5 presents the heart rate histories of two subjects over the entire course of progressively increasing LBNP and the subsequent venting of the chamber. While in the case of subject #5, as shown in Figure 5 the heart rate increases significantly. In the case of subject #9 there is next to no discernible change during the progressive LBNP increases, as shown in Figure 2B.

### Cuff-based Systolic & Diastolic Blood Pressure Changes

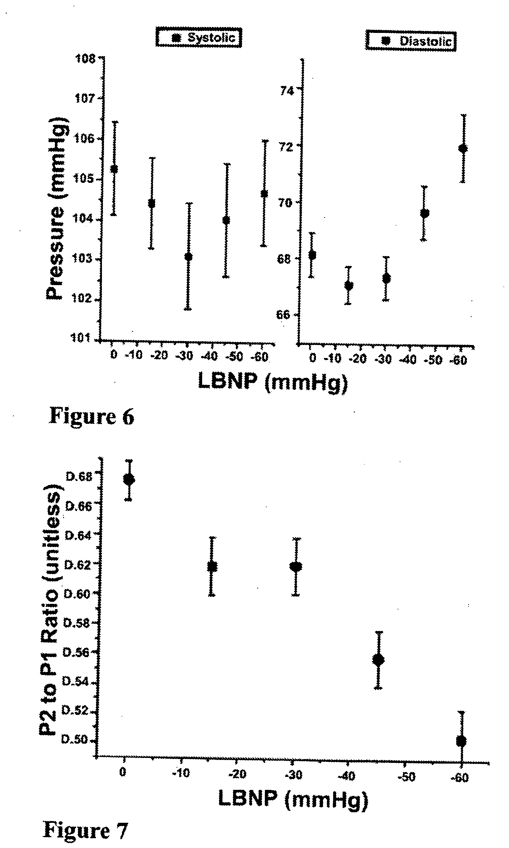

In regard to the systolic blood pressure recorded with the automatic cuff, next to no correlation with LBNP was determined. As illustrated in Figure 6, the diastolic pressure showed a modest increase with increasing LBNP. They are in contrast to those reported in publication 2, which reported a decline in systolic pressureof 18 mmHg with increasing LBNP (same range as used here) and next to no change in diastolic pressure in a cohort of subjects with an average age of 15 more years than the subjects studied here.

In contrast to the cuff results the pulse decomposition analysis (PDA) parameter that is equivalent to systolic pressure, the P2:P1 ratio did show a statistically significant decrease with LBNP as presented in Figure 7. This PDA parameter is determined by taking the ratio of the amplitude of the renal reflection pulse (#2 pulse) to the amplitude of the primary left ventricular ejection pulse (#1 pulse).

However, while the average effect had statistical significance, no consistent trend was recorded across all subjects, a result verified in other studies that have found that systolic pressure is not a reliable predictor for central blood loss.

In regard to the discrepancy between the automatic cuff results obtained in publication 2 and in this study it is important to note that the blood pressure ranges reported here are very small; on the order of 5 and 8 mmHg in the case of the systolic and diastolic pressures, respectively. It is very difficult to resolve blood pressure trends within such small limits with automatic brachial cuffs due to their instrumental uncertainties and differences in proprietary algorithms. As an example, one study that compared the performance of brachial cuffs and catheters revealed standard deviations (SD) on the order of 12 mmHg with essentially zero bias in the case of systolic blood pressures and variations on the order of 12 mmHg as well as a positive bias of 10 mmHg in the case of diastolic pressure measurements.

### Pulse Pressure Changes

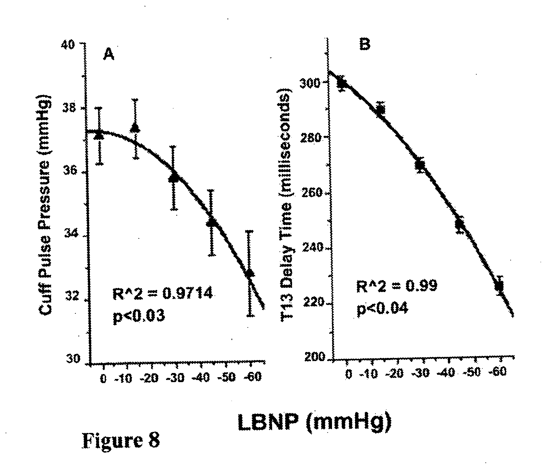

Figure 8A presents the overall pulse pressure results of the automatic pressure cuff as function of LBNP while Figure 8B presents the overall results of the pulse pressure equivalent PDA arterial pulse parameter T1 -T3, which is the time difference between the arrival of the primary left ventricular ejection pulse (pulse #1 ) and the arrival of the iliac reflection (pulse #3).

It is seen that while both measurement methods resolve the effect at a statistically significant level, the ability to make a real-time determination of the onset of hemorrhage in individual cases using the two methods differs greatly.

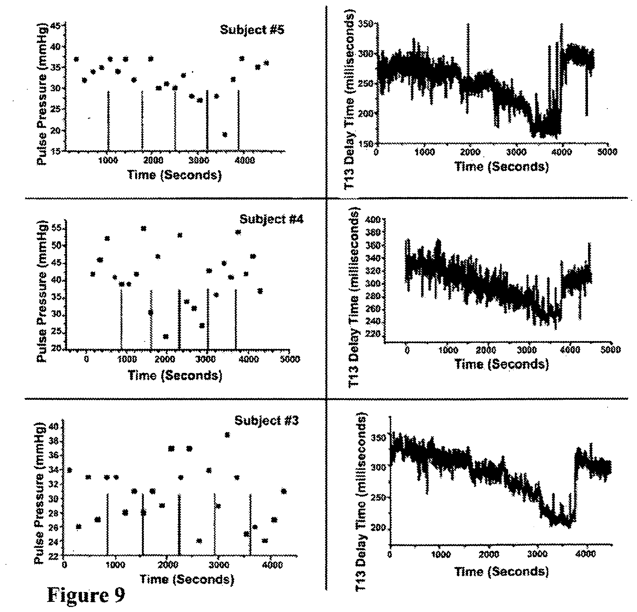

Figure 9 presents side-by-side comparisons of pulse pressures obtained with the automatic cuff (left column graphs) and the histories of the T1 -T3 parameter over the course of LBNP session, (right column graphs). The right panels present the simultaneously obtained T1 -3 delay times between the primary left- ventricular ejection pulse and the iliac reflection pulse recorded on the subjects middle member of the middle digit. The top row presents graphs of the most clearly resolved change in pulse pressures determined with the automatic cuff, left, and of the change in the T1 -T3 parameter, right, for subject #5. The center row presents the same for subject #9. This is the same subject whose heart rate did not respond to the LBNP changes, which were presented in Figure 5. Similarly, the cuff-based pulse pressures show no discernible trend. The situation is quite different with regard to the T1 -T3 parameter whose temporal evolution reveals the plateaus of the individual LBNP stages. The bottom row presents the same comparison of results for subject #3.

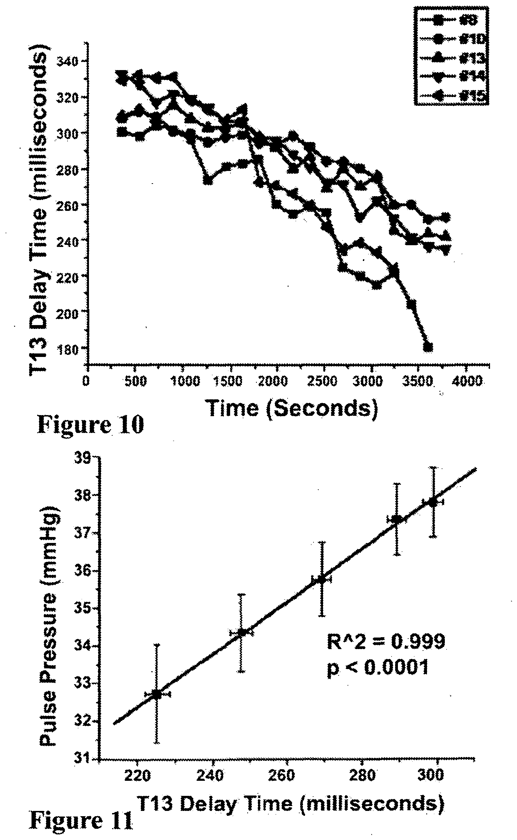

Every one of the fifteen subjects studied exhibited statistically significant decreases in T1 -T3 as a function of LBNP. Figure 10 displays fifteen- second averages of T1 -T3 for five other subjects not presented so far within a minute of the time the blood pressure cuff took its measurement. Given the results presented so far it is clear that a comparable presentation of cuff-derived pulse pressures would be meaningless.

This study also presents a validation of the T1-T3 parameter as being the arterial pulse parameter that correlates with pulse pressure. Figure 11 presents a linear correlation of the T1 -T3 parameter and the cuff-derived pulse pressure, both of which appear to have a non-linear dependence on central blood loss based on the results presented in Figure 8.

If they are indeed equivalent, their correlation should be linear, which it is at a high level of significance. In addition the correlation provides a statistically relevant conversion factor for relating T1 -T3 values to pulse pressures for individuals with T1 -T3 values in the neighborhood of 300 milliseconds at resting blood pressures.

Figures 10 and 11 show graphs illustrating results of tests using the disclosed system. Figure 10 illustrates a comparison of the pulse line shapes obtained with a central line catheter with simultaneously obtained derivative pulse line shapes using the disclosed system. Of particular interest is the size of the renal reflection. The relative amplitudes of systolic peak and renal reflection obtained centrally match those obtained peripherally. Also illustrated in this Figure is a graph showing that in more than half the patients in the study there were periodic significant variations in systolic blood pressure as measured using the disclosed system. The disclosed data extends for four minutes and shows a series of drops, some lasting more than half a minute and extending over 20 or so heartbeats. The catheter observation time window missed the majority of these decreases because the protocol entry allowed 18 seconds of quiet observation at the renal artery region.

### Apparatus for use in accordance with the present invention

Figure 12 shows the apparatus configuration in which a detector, or sensor unit, 1200 sends an analog signal to a computer 1210. The analog signal is converted to a digital signal in the analog/digital converter 1212. As is typical of computers, computer 1210 include a processor (cpu) 1214 and computer memory 1216. The computer system also include a blood volume detector 1218, a data comparison unit 1220, time differential detector 1222, diagnostic support unit 1224, and a time change monitor 1226 for monitoring time changes between the first pulse detected and the second pulse detected. The second pulse corresponds to the third peak, as previous described. The data output generator 1230 sends the data to the desired device. By displaying results, data, and the like on a display member 1230, the data can be viewed by a user. It should be noted that the communication between the sensor 1200, computer 1210 and display 1230, or other output device, can be wired or wireless. Communication methods between the electronic devices of this type are well known in the art.

### Conclusions

The results presented support that pulse pressure is a reliable indicator of central hypovolemia, decreasing early and with progressing decreasing magnitude as central blood loss increases in a non-linear manner. Equally important is the result that utilization of the technology of the present invention with use of the PDA algorithm provides a means to monitor pulse pressure, and therefore the progression of hemorrhage, reliably in a real-time fashion.

The results of this study also support the hypothesis that pulse pressure and the T1 -T3 parameter of the PDA algorithm are equivalent. The difference in the arrival times of the primary arterial pulse that is the left ventricular ejection and the iliac reflection pulse is determined by the differential velocities with which both pulses propagated along their arterial paths. In the case of the iliac reflection the path length is longer than that of the primary pulse by almost twice the length of the torso. More importantly, the pulse's arterial propagation velocities are pressure dependent, a relationship long known through the Moens-Korteweg equation, as noted in publication 5. One central insight is that both pulses travel at different velocities because their pressure amplitudes are different, the iliac reflection pulse amplitude, which is determined by the reflection coefficient of the iliac reflection site, being on the order of 40% of pulse pressure. Both pulses therefore load the arterial wall differently during their arterial travel, as a result of which their propagation velocities are different. The second insight is that, because the pressure/velocity response curve is non-linear - a result known since the 1960s based on Anliker's work, (publication 6) - both pulses accelerate and decelerate at different rates as the pressure rises and falls. The primary pulse experiences the highest changes in velocity as a function of changes in blood pressure because it is subject to the steepest section of the pressure/velocity response curve, while the iliac pulse, "running" at much lower pressure, changes velocity much more gradually. Changes in the time of arrival therefore then reflect changes in the differential arterial pressure that the two pulses experience. While this differential pressure is not exactly pulse pressure - pulse pressure being the difference between the full pulse arterial pulse height and the diastolic pressure floor - it represents about 60% of it, assuming the previously stated iliac reflection coefficient. More importantly, as the results of this study indicate, it tracks the changes in pulse pressure at a very high time resolution.

This time resolution is a significant benefit of measuring T1 -T3 over pulse pressure because it offers higher resolution. The results indicate the equivalence of a change of about 200 milliseconds in T1-T3 to a variation of about 8 mmHg in pulse pressure over the entire range of a simulated central blood loss in excess of 1 liter for this cohort of fit and relatively young subjects. Given the uncertainties in determining pulse pressures with automated cuffs, the likelihood of resolving variations on the order of a single mmHg as central blood loss commences is remote at best. In comparison, the possibility of resolving changes in T1 -T3 on the order of 10-20 milliseconds as central blood loss progresses is quite feasible based on the results presented.

One could argue that the pulse pressure changes to be expected might be larger, given the results of the Convertino study (2), which recorded average changes in' pulse pressure of 18 mmHg. While this change in pulse pressure would likely still be difficult to resolve reliably with standard BP equipment the difference in the studies' results may point out another issue. As stated above, one reason for the difference in pulse pressure variations may be the difference in age and fitness of the respective participants. The average age of the subjects in the Convertino study was 42 years, as compared to 24.3 years in this study. Likewise, the average resting systolic blood pressure was 129 mmHg in the Convertino study as compared to 105 mmHg in this study, while the average pulse pressure was 51 mmHg compared to 37 mmHg. It is well known that pulse pressure rises as arterial walls harden, such as due to normal aging or pathological influences. Similar differences in subject groups differentiated by age or vascular health, such as diabetic patients, have been observed in the T1 -T3 parameter.

Young athletes typically have T1- 3 delay times in the range of 300 milliseconds while vascularly challenged subjects have T1-T3 delay times in the low 200 millisecond range, at comparable blood pressures and heart rates. The obvious explanation is that delay times between the two pulses shorten as pulse propagation velocities increase with hardening arterial walls. Since the subject group studied here is arguably more representative of today's armed forces membership, one of the primary target populations for early hemorrhagic shock detection, the results of this study further buttress the relevance of utilizing T1 -T3 as an early indicator in a combat environment because it is this group that likely will have the lowest resting pulse pressures and, correspondingly, largest T1 -T3 values.

### Broad Scope of the Invention

Although the present invention has been fully described in conjunction with several embodiments thereof with reference to the accompanying drawings, it is to be understood that various changes and modifications may be apparent to those skilled in the art. The present invention is defined by the scope of the appended claims.

The limitations in the claims are to be interpreted broadly based on the language employed in the claims and not limited to examples described in the present specification or during the prosecution of the application, which examples are to be construed as non-exclusive. For example, in the present disclosure, the term "preferably" is non-exclusive and means "preferably, but not limited to." In this disclosure and during the prosecution of this application, means-plus-function or step plus-function limitations will only be employed where for a specific claim limitation all of the following conditions are present in that limitation: a) "means for" or "step for" is expressly recited; b) a corresponding function is expressly recited; and c) structure, material or acts that support that structure are not recited. In this disclosure and during the prosecution of this application, the terminology "present invention" or "invention" may be used as a reference to one or more aspects within the present disclosure. The language present invention or invention should not be improperly interpreted as an identification of criticality, should not be improperly interpreted as applying across all aspects or embodiments (i.e., it should be understood that the present invention has a number of aspects and embodiments). In this disclosure and during the prosecution of this application, the terminology "embodiment" can be used to describe any aspect, feature, process or step, any combination thereof, and/or any portion thereof, etc. In some examples, various embodiments may include overlapping features.

In this disclosure, the following abbreviated terminology may be employed:"e.g." which means "for example".

## Claims

1. A diagnostic support apparatus comprising:
- a detecting means (1200) for detecting an analog signal of a pulse waveform from a detecting position of an animate being and transmitting said analog signal;
- a processor (1210), said processor (1210) having:
means for receiving said analog signal;
a converter to convert said analog signal to a digital signal;
an algorithm for locating pulses within said waveform and locating a center of each pulse;
- a time differential detector that is **characterized in** being adapted for measuring the time difference between the arrival of a first pulse (100), said first pulse being the primary left ventricular ejection pulse and the arrival of a second pulse, said second pulse being the iliac reflection pulse, said first pulse (100) and said second pulse (104) being pulses whose changes in time difference corresponds to changes in blood volume due to hypervolemia or hypovolemia in said animate being, said first pulse (100) corresponding to the first peak on a pulse wave form and the second pulse (104) corresponding to a different peak on the pulse wave form.

2. The diagnostic support apparatus of claim 1, **characterized in that** said detecting means (1200) detects said first peak on the pulse wave form and a third peak on the pulse wave form and wherein said first pulse (100) corresponds to said first peak and said second pulse(104) corresponds to said third peak.

3. The diagnostic support apparatus of claim 1, **characterized in that** said apparatus further comprising data storage unit (1228) for storing measured time differences between said first pulse (100) and said second pulse(104) for a period of time,
said period of time being at least about fifteen seconds and wherein said detecting means (1200) is adapted to measure said pulse waveform in real time, or
said period of time being a predetermined continuous period of time, said continuous period of time being sufficient to produce statistically significant arterial pulse parameter data represented by said time difference or
said period of time being a plurality of random or predetermined discontinuous time intervals over a period of at least a half hour.

4. The diagnostic support apparatus according to any one of the preceding claims, **characterized in that** said apparatus further comprises:
- a diagnostic support unit (1224) to create diagnostic support information from data stored in said data storage unit (1228);
said diagnostic support unit (1224) calculates a characteristic value from said time difference between the arrival of said first pulse (100) and the arrival of said second pulse(104), contained in the diagnostic information and creates diagnostic support information based on the characteristic value calculated by the characteristic value calculation means, the apparatus being **characterized in that** it comprises detection means for detecting changes in the characteristic time differential between the first pulse (100) and the second pulse (104) over a time period sufficient to indicate blood volume changes.

5. The diagnostic support apparatus according to any one of the preceding claims, **characterized in that** said apparatus further comprising a data comparison unit (1220) to compare said time difference with historical data from said data storage unit (1228) indicative of blood volume, said historical data comprising time difference data for said animate being and/or a comparable animate being group.

6. A diagnostic support method of providing diagnostic support comprising:
- applying a detecting means to an artery of an animate being,
- generating an analog signal of a pulse waveform,
- at a processor converting said analog signal to a digital signal, locating pulses within said waveform and locating a center peak of each pulse where the processor is **characterized in** being adapted for determining the time difference between the arrival of a first pulse (100), said first pulse being the primary left ventricular ejection pulse, and the arrival of a second pulse (104), said second pulse being the iliac reflection pulse, said first pulse (100) and said second pulse(104) being pulses whose changes in time difference correspond to changes in blood volume due to hypervolemia or hypovolemia in said animate being,
- storing said data in a data storage unit (1228),
- creating diagnostic support information (1224) using the stored data in said data storage unit (1228).

7. The diagnostic support method of claim 6 **characterized in that** the method further comprising the step of measuring said time difference for a predetermined continuous period of time,
said period of time being sufficient to produce statistically significant arterial pulse parameter data or
said period of time being a plurality of random or predetermined discontinuous time intervals over a period of at least a half hour.

8. A computerized diagnostic support method for real time determination of blood volume in an animate the method comprising the steps of:
- monitoring a pulse waveform and, using a processor (1210), locating pulses and their peaks within said waveform, where the method is **characterized in** comprising the steps of:
- monitoring the time difference between the arrival of a first pulse (100), said first pulse being the primary left ventricular ejection pulse, and the arrival of a second pulse (104), said second pulse being the iliac reflection pulse, said first pulse (100) and said second pulse (104) being pulses whose time difference corresponds to blood volume in said animate being,
- generating time difference data, and
- storing said time difference data in a data storage unit (1228),
- comparing current data with stored data to determine change in blood volume, said blood volume indicating a change in blood pressure.

## Patentansprüche

1. Diagnoseunterstützungsvorrichtung, umfassend:
- eine Erfassungseinrichtung (1200) zum Erfassen eines analogen Signals einer Pulswellenform von einer Erfassungsposition eines lebenden Wesens und zum Übertragen des analogen Signals;
- einen Prozessor (1210), wobei der Prozessor (1210) aufweist:
- Mittel zum Empfangen des analogen Signals;
- einen Konverter zum Konvertieren des analogen Signals in ein digitales Signal;
- einen Algorithmus zum Lokalisieren von Impulsen innerhalb der Wellenform und zum Lokalisieren einer Mitte jedes Impulses;
- einen Zeitdifferenzdetektor, der **dadurch gekennzeichnet ist, dass** er zum Messen der Zeitdifferenz zwischen dem Eintreffen eines ersten Impulses (100), wobei der erste Impuls der primäre linksventrikuläre Auswurfimpuls ist, und dem Eintreffen eines zweiten Impulses, wobei der zweite Impuls der iliaker Reflexionspuls ist, wobei der erste Impuls (100) und der zweite Impuls (104) Impulse sind, deren Änderungen der Zeitdifferenz Änderungen des Blutvolumens aufgrund von Hypervolämie oder Hypovolämie in dem belebten Körper entsprechen, wobei der erste Impuls (100) dem ersten Spitze entspricht einer Pulswellenform und der zweite Puls (104) einer anderen Spitze der Pulswellenform entspricht.

2. Diagnosestützvorrichtung nach Anspruch 1, **dadurch gekennzeichnet, dass** die Erfassungseinrichtung (1200) die erste Spitze in der Pulswellenform und eine dritte Spitze in der Pulswellenform erfasst, und wobei der erste Impuls (100) der ersten Spitze entspricht und der zweite Impuls (104) der dritten Spitze entspricht.

3. Diagnosestützvorrichtung nach Anspruch 1, **dadurch gekennzeichnet, dass** die Vorrichtung ferner eine Datenspeichereinheit (1228) zum Speichern gemessener Zeitdifferenzen zwischen dem ersten Impuls (100) und dem zweiten Impuls (104) für eine Zeitspanne aufweist,
wobei die Zeitspanne mindestens etwa fünfzehn Sekunden beträgt und wobei die Erfassungseinrichtung (1200) dazu ausgelegt ist, die Pulswellenform in Echtzeit zu messen, oder
wobei der Zeitraum ein vorbestimmter kontinuierlicher Zeitraum ist, wobei der kontinuierliche Zeitraum ausreichend ist, um statistisch signifikante arterielle Impulsparameterdaten zu erzeugen, die durch die Zeitdifferenz repräsentiert werden oder
wobei der Zeitraum mehrere zufällige oder vorbestimmte diskontinuierliche Zeitintervalle über einen Zeitraum von mindestens einer halben Stunde ist.

4. Diagnoseunterstützungsvorrichtung nach einem der vorhergehenden Ansprüche, **dadurch gekennzeichnet, dass** die Vorrichtung ferner umfasst:
- eine Diagnoseunterstützungseinheit (1224) zum Erzeugen von Diagnoseunterstützungsinformationen aus Daten, die in der Datenspeichereinheit (1228) gespeichert sind;
die Diagnoseunterstützungseinheit (1224) berechnet einen charakteristischen Wert aus der Zeitdifferenz zwischen dem Eintreffen des ersten Impulses (100) und dem Eintreffen des zweiten Impulses (104), der in der Diagnoseinformation enthalten ist, und Charakteristik Diagnosestützinformation erzeugt basierend auf der durch die Kennwertberechnungseinrichtung berechneter Wert, wobei die Vorrichtung **dadurch gekennzeichnet ist, dass** sie eine Erfassungseinrichtung zum Erfassen von Änderungen in der charakteristischen Zeitdifferenz zwischen dem ersten Impuls (100) und dem zweiten Impuls (104) über eine Zeitdauer aufweist, die ausreicht, um Blutvolumenänderungen anzuzeigen.

5. Diagnosestützvorrichtung nach einem der vorhergehenden Ansprüche, **dadurch gekennzeichnet, dass** die Vorrichtung ferner eine Datenvergleichseinheit (1220) umfasst zum Vergleichen der Zeitdifferenz mit historischen Daten aus der Datenspeichereinheit (1228) die das Blutvolumen anzeigen, wobei die historischen Daten Zeitdifferenzdaten für das belebte Wesen und / oder eine vergleichbare lebende Wesengruppen umfassen.

6. Diagnoseunterstützungsverfahren zum Bereitstellen von Diagnoseunterstützung, umfassend:
- Anwenden eines Nachweismittels auf eine Arterie eines lebenden Wesens,
- Erzeugen eines analogen Signals einer Pulswellenform,
- bei einem Prozessor, der das analoge Signal in ein digitales Signal umwandelt, Lokalisieren von Impulsen innerhalb der Wellenform und Lokalisieren einer Mittenspitze jedes Impulses, wobei der Prozessor **dadurch gekennzeichnet ist, dass** er die Zeitdifferenz zwischen dem Eintreffen eines ersten Impulses (100) bestimmt, wobei der erste Impuls der primäre linksventrikuläre Auswurfimpuls ist und das Eintreffen eines zweiten Impulses (104), der zweite Impuls der iliakale Reflexionsimpuls ist, wobei der erste Impuls (100) und der zweite Impuls (104) Impulse sind, deren Änderungen in der Zeitunterschied den Änderungen in den Blutvolumens entsprechen aufgrund von Hypervolämie oder Hypovolämie in dem lebende Wesen,
- Speichern der Daten in einer Datenspeichereinheit (1228),
- Erzeugen von Diagnoseunterstützungsinformationen (1224) unter Verwendung der in der Datenspeichereinheit (1228) gespeicherten Daten.

7. Diagnoseunterstützungsverfahren nach Anspruch 6, **dadurch gekennzeichnet, dass** das Verfahren ferner den Schritt des Messens der Zeitdifferenz für eine vorbestimmte kontinuierliche Zeitdauer umfasst,
wobei der Zeitraum ausreichend ist, um statistisch signifikante arterielle Impulsparameterdaten zu erzeugen oder
wobei der Zeitraum mehrere zufällige oder vorbestimmte diskontinuierliche Zeitintervalle über einen Zeitraum von mindestens einer halben Stunde ist.

8. Computergestütztes diagnostisches Unterstützungsverfahren zur Echtzeitbestimmung des Blutvolumens in einem lebenden Wesen, wobei das Verfahren die folgenden Schritte umfasst:
- Überwachen einer Impulswellenform und Lokalisieren von Impulsen und ihrer Spitzen innerhalb der genannten Wellenform unter Verwendung eines Prozessors (1210), wobei das Verfahren **dadurch gekennzeichnet ist, dass** es die folgenden Schritte umfasst:
- Überwachen der Zeitdifferenz zwischen dem Eintreffen eines ersten Impulses (100), wobei der erste Impuls der primäre linksventrikuläre Auswurfimpuls ist, und dem Eintreffen eines zweiten Impulses (104), wobei der zweite Impuls der iliakale Reflexionsimpuls ist, wobei der erste Impuls (100) und der zweite Impuls (104) Impulse sind, deren Zeitdifferenz dem Blutvolumen in dem lebende Wesen entspricht,
- Erzeugen von Zeitdifferenzdaten und
- Speichern der Zeitdifferenzdaten in einer Datenspeichereinheit (1228),
- Vergleichen von aktuellen Daten mit gespeicherten Daten, um die Änderung des Blutvolumens zu bestimmen, wobei das Blutvolumen eine Änderung des Blutdrucks anzeigt.

## Revendications

1. Un dispositif d'assistance au diagnostic comprenant
- un moyen de détection (1200) pour détecter un signal analogique d'une forme d'onde de pouls d'une position de détection d'un être vivant et pour transmettre le signal analogique;
- un processeur (1210), le processeur (1210) présentant les caractéristiques suivantes:
- des moyens pour recevoir ledit signal analogique;
- un convertisseur pour convertir ledit signal analogique en un signal numérique;
- un algorithme pour localiser des impulsions dans la forme d'onde et localiser un centre de chaque impulsion;
- un détecteur de différence de temps **caractérisé en ce qu'**il est adapté pour mesurer la différence de temps entre l'arrivée d'une première impulsion (100), ladite première impulsion étant l'impulsion d'éjection ventriculaire gauche principale et l'arrivée d'une seconde impulsion, ladite seconde impulsion étant l'impulsion de réflexion iliaque, ladite première impulsion (100) et ladite seconde impulsion (104) étant des impulsions dont les variations de la différence de temps correspondent à des modifications du volume sanguin dues à une hypervolémie ou une hypovolémie chez ladite créature vivante, ladite première impulsion (100) correspondant à avec le premier pic sur une forme d'onde de pouls et la seconde impulsion (104) correspond à un autre pic sur la forme d'onde de pouls.

2. Dispositif d'assistance de diagnostic selon la revendication 1, **caractérisé en ce que** les moyens de détection (1200) détectent le premier pic sous la forme d'onde de pouls et un troisième pic sous la forme d'onde de pouls et dans lequel la première impulsion (100) correspond au premier pic et à la seconde impulsion (104) correspond au troisième pic.

3. Dispositif d'assistance de diagnostic selon la revendication 1, **caractérisé en ce que** le dispositif comprend en outre une unité de stockage de données (1228) pour stocker des différences de temps mesurées entre la première impulsion (100) et la seconde impulsion (104) pendant une période de temps; la période de temps étant d'au moins environ quinze secondes et dans lequel le moyen de détection (1200) est adapté pour mesurer la forme d'onde du pouls en temps réel, ou
ladite période de temps étant une période de temps continue prédéterminée, ladite période de temps continue étant suffisante pour produire des données de paramètre d'impulsion artérielle statistiquement significatives représentées par
ladite différence de temps ou
la période de temps étant un nombre d'intervalles de temps discontinus aléatoires ou prédéterminés pendant une période d'au moins une demi-heure.

4. Dispositif d'assistance au diagnostic selon l'une quelconque des revendications précédentes, **caractérisé en ce que** le dispositif comprend en outre:
- une unité de support de diagnostic (1224) pour créer des informations de support de diagnostic des données stockées dans l'unité de stockage de données (1228);
dans lequel l'unité de support de diagnostic (1224) calcule une valeur caractéristique à partir de la différence de temps entre l'arrivée de la première impulsion (100) et l'arrivée de la seconde impulsion (104), qui est dans les informations de diagnostic et crée des informations de support de diagnostic sur la base du valeur caractéristique calculée par le moyen de calcul de valeur caractéristique, le dispositif étant **caractérisé en ce qu** 'il comprend un moyen de détection pour détecter des changements dans la différence de temps caractéristique entre la première impulsion (100) et la seconde impulsion (104) pendant une période suffisante pour indiquer les changements du volume sanguin.

5. Dispositif d'assistance de diagnostic selon l'une des revendications précédentes, **caractérisé en ce que** le dispositif comprend en outre une unité de comparaison de données (1220) destinée à comparer la différence de temps avec des données historiques provenant de l'unité de stockage de données (1228) qui indiquent le volume de sang, lesdites données historiques comprennent des données de différence de temps pour ladite entité vivante et / ou un groupe similaire d'êtres vivants.

6. Une méthode d'aide au diagnostic pour fournir une aide au diagnostic, comprenant:
- appliquer un moyen de détection sur une artère d'un être vivant,
- générer un signal analogique d'une forme d'onde de pouls,
- avec un processeur convertisant ledit signal analogique en un signal numérique, localisant des impulsions dans ladite forme d'onde et localisant un pic central de chaque impulsion, le processeur étant **caractérisé en ce qu'**il est adapté pour déterminer la différence de temps entre l'arrivée de une première impulsion (100), la première impulsion étant l'impulsion d'éjection du ventricule gauche primaire et l'arrivée d'une seconde impulsion (104), la seconde impulsion étant l'impulsion de réflexion iliaque, la première impulsion (100) et la seconde impulsion (104) étant des impulsions dont les variations de la différence de temps correspondent à des modifications du volume sanguin dues à une hypervolémie ou à une hypovolémie chez l'être vivant,
- stocker les données dans une unité de stockage de données (1228),
- créer des informations de support de diagnostic (1224) en utilisant les données stockées dans l'unité de stockage de données (1228).

7. Procédé d'aide au diagnostic selon la revendication 6, **caractérisé en ce que** le procédé comprend en outre l'étape consistant à mesurer la différence de temps pendant une période de temps continue prédéterminée,
la période de temps étant suffisante pour produire des données de paramètre d'impulsion artérielle statistiquement significatives ou
la période de temps étant un nombre d'intervalles de temps discontinus aléatoires ou prédéterminés pendant une période d'au moins une demi-heure.

8. Un procédé automatisé d'aide au diagnostic pour déterminer le volume sanguin en temps reel chez un être vivant, le procédé comprenant les étapes suivantes:
- surveiller une forme d'onde de pouls et, à l'aide d'un processeur (1210), localiser les impulsions et leurs pics dans la forme d'onde, le procédé étant **caractérisé en ce qu'**il comprend les étapes suivantes:
- surveiller la différence de temps entre l'arrivée d'une première impulsion (100), ladite première impulsion étant l'impulsion d'éjection du ventricule gauche primaire et l'arrivée d'une seconde impulsion (104), ladite seconde impulsion étant l'impulsion de réflexion iliaque, et lesdites première impulsion (100) et seconde impulsion (104) étant des impulsions dont la différence de temps correspond au volume de sang dans l'être vivant,
- générer des données de décalage horaire, et
- stocker les données de différence de temps dans une unité de stockage de données (1228),
- en comparant les données actuelles aux données stockées pour déterminer une variation du volume sanguin, le volume sanguin indiquant une variation de la pression artérielle.
